(19) Europäisches Patentamt
European Patent Office
Office européen des brevets

(11) **EP 1 196 433 B1**

(12) **EUROPÄISCHE PATENTSCHRIFT**

(45) Veröffentlichungstag und Bekanntmachung des Hinweises auf die Patenterteilung:
**27.09.2006 Patentblatt 2006/39**

(21) Anmeldenummer: **00943971.2**

(22) Anmeldetag: **03.07.2000**

(51) Int Cl.:
*C07K 7/56* (2006.01)          *C07K 7/64* (2006.01)
*A61K 38/04* (2006.01)          *A61P 7/02* (2006.01)

(86) Internationale Anmeldenummer:
**PCT/EP2000/006188**

(87) Internationale Veröffentlichungsnummer:
**WO 2001/005810 (25.01.2001 Gazette 2001/04)**

(54) **CYCLISCHE PEPTIDDERIVATE ALS INHIBITOREN DES INTEGRINS ALPHA v BETA 6**

CYCLIC PEPTIDE DERIVATIVES AS INHIBITORS OF INTEGRIN ALPHA v BETA 6

DERIVES PEPTIDIQUES CYCLIQUES SERVANT D'INHIBITEURS DE L'INTEGRINE ALPHA v BETA 6

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **15.07.1999 DE 19933173**

(43) Veröffentlichungstag der Anmeldung:
**17.04.2002 Patentblatt 2002/16**

(73) Patentinhaber: **Merck Patent GmbH**
**64293 Darmstadt (DE)**

(72) Erfinder:
 • **JONCZYK, Alfred**
  **D-64295 Darmstadt (DE)**
 • **DIEFENBACH, Beate**
  **D-81739 München (DE)**
 • **GOODMAN, Simon**
  **D-64347 Griesheim (DE)**
 • **GROTH, Ulrich**
  **D-78464 Konstanz (DE)**
 • **ZISCHINSKY, Gunther**
  **D-78462 Konstanz (DE)**

(56) Entgegenhaltungen:
  **WO-A-00/37487**

 • **M-L VALERO ET AL.: "Solid-mediated cyclization of head-to-tail peptides; problems associated with side-chain anchoring" TETRAHEDRON LETTERS., Bd. 37, Nr. 24, 1996, Seiten 4229-4232, XP002155911 ELSEVIER SCIENCE PUBLISHERS, AMSTERDAM., NL ISSN: 0040-4039**
 • **T JACKSON ET AL.: " Arginine-glycine-aspartic acid-specific binding by foot-and-mouth disease viruses to the purified integrin alphaVbeta3 in vitro" JOURNAL OF VIROLOGY., Bd. 71, Nr. 11, November 1997 (1997-11), Seiten 8357-8361, XP002136308 THE AMERICAN SOCIETY FOR MICROBIOLOGY., US ISSN: 0022-538X**
 • **S KRAFT ET AL.: "Definition of an unexpected ligand recognition motif for alphaVbeta6 integrin" JOURNAL OF BIOLOGICAL CHEMISTRY., Bd. 274, Nr. 4, 22. Januar 1999 (1999-01-22), Seiten 1979-1985, XP002136307 AMERICAN SOCIETY OF BIOLOGICAL CHEMISTS, BALTIMORE, MD., US ISSN: 0021-9258 in der Anmeldung erwähnt**
 • **GILON ET AL: 'Backbone cyclization: A new method for conferring conformational constraint on peptides' BIOPOLYMERS Bd. 31, Nr. 6, 1991, Seiten 745 - 750**

Bemerkungen:
  Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neuartige Peptidderivate der Formel I

$$\text{Cyclo-(Arg-X}^1\text{-Asp-X}^2\text{-X}^3\text{-X}^4\text{-X}^5\text{-X}^6\text{-R}^1) \qquad \text{I}$$

worin

$X^1$    Ser, Gly oder Thr,
$X^2$    Leu, Ile, Nle, Val oder Phe,
$X^3$    Asp, Glu, Lys oder Phe,
$X^4$    Gly, Ala oder Ser,
$X^5$    Leu, Ile, Nle, Val oder Phe,
$X^6$    Arg, Har oder Lys,
$R^1$    einen oder mehrere ω-Aminocarbonsäurerest(e), wobei der oder die ω-Aminocarbonsäurerest(e) eine Länge von 500 bis 2500 pm aufweisen,

bedeuten,
wobei
die D- als auch die L-Formen der optisch aktiven Aminosäurereste eingeschlossen sind,
sowie deren physiologisch unbedenklichen Salze und Solvate.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die erfindungsgemäßen Verbindungen und ihre Salze bei guter Verträglichkeit sehr wertvolle pharmakologische Eigenschaften besitzen.

Die erfindungsgemäßen Peptide können als wirksame Inhibitoren des $\alpha_v\beta_6$ Integrin-Rezeptors und somit zur Behandlung verschiedener Krankheiten und pathologischer Befunde eingesetzt werden.

Andere Inhibitoren des Integrins $\alpha_v\beta_6$ sind in der DE 19858857 und von S.Kraft et al. in J. Biol. Chem.274, 1979-85 (1999) beschrieben. Die erfindungsgemäßen Verbindungen sind in bezug auf die genannte Anmeldung als Auswahlerfindung zu betrachten.

Integrine gehören zu der Familie von heterodimeren Klasse I - Transmembran-Rezeptoren, die in zahlreichen Zell-Matrix- bzw. Zell-Zell-Adhäsionsvorgängen eine wichtige Rolle spielen (Tuckwell et al., 1996, Symp. Soc. Exp. Biol. 47). Sie können grob in drei Klassen eingeteilt werden: die $\beta_1$-Integrine, die Rezeptoren für die extrazelluläre Matrix darstellen, die $\beta_2$-Integrine, welche auf Leukozyten aktivierbar sind und während inflammatorischen Prozessen "getriggert" werden, sowie die $\alpha_v$-Integrine, die die Zellantwort bei Wundheilungs- und anderen pathologischen Prozessen beeinflussen (Marshall and Hart, 1996, Semin. Cancer Biol. 7, 191).

Die Integrine $\alpha_5\beta_1$, $\alpha_{IIb}\beta_3$, $\alpha_8\beta_1$, $\alpha_v\beta_1$, $\alpha_v\beta_3$, $\alpha_v\beta_5$, $\alpha_v\beta_8$ und $\alpha_v\beta_6$ binden alle an die Arg-Gly-Asp (RGD) Peptidsequenz in natürlichen Liganden, wie z.B. Fibronektin oder Vitronektin. Lösliche RGD-haltige Peptide vermögen die Interaktion jedes dieser Integrine mit dem entsprechenden natürlichen Liganden zu inhibieren. $\alpha_v\beta_6$ ist ein relativ seltenes Integrin (Busk et al., 1992 J. Biol. Chem. 267(9), 5790), das bei Reperaturvorgängen in Epithelgewebe vermehrt gebildet wird und die natürlichen Matrixmoleküle Fibronectin und Tenascin bevorzugt bindet (Wang et al., 1996, Am. J. Respir. Cell Mol. Biol. 15(5), 664). Die physiologischen und pathologischen Funktionen von $\alpha_v\beta_6$ sind noch nicht genau bekannt, es wird jedoch vermutet, daß dieses Integrin bei physiologischen Vorgängen und Erkrankungen (z. B. Entzündungen, Wundheilung, Tumore), bei denen epitheliale Zellen beteiligt sind, eine wichtige Rolle spielt. So wird $\alpha_v\beta_6$ auf Keratinozyten in Wunden exprimiert (Haapasalmi et al., 1996, J. Invest. Dermatol. 106(1), 42), woraus anzunehmen ist, daß neben Wundheilungsprozessen und Entzündungen auch andere pathologische Ereignisse der Haut, wie z. B. Psoriasis, durch Agonisten oder Antagonisten des besagten Integrins beeinflußbar sind. Ferner spielt $\alpha_v\beta_6$ im Atemwegsepithel eine Rolle (Weinacker et al., 1995, Am. J. Respir. Cell Mol. Biol. 12(5), 547), so daß entsprechende Agonisten / Antagonisten dieses Integrins bei Atemwegserkrankungen, wie Bronchitis, Asthma, Lungenfibrosen und Atemwegstumoren erfolgreich eingesetzt werden könnten. Letztlich ist bekannt, daß $\alpha_v\beta_6$ auch im Darmepithel eine Rolle spielt, so daß entsprechende Integrin-Agonisten/-Antagonisten bei der Behandlung von Entzündungen, Tumoren und Wunden des Magen/Darmtraktes Verwendung finden könnten.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Es bestand somit die Aufgabe, neben den bisher bekannten natürlichen hochmolekularen Liganden und Antikörpern, die therapeutisch und diagnostisch schwer handhabbar sind, potente, spezifische bzw. selektive niedermolekulare Liganden für $\alpha_v\beta_6$, vorzugsweise Peptide, zu finden, die für die genannten therapeutischen Gebiete aber auch als Diagnostikum oder Reagenz verwendet werden können.

Es wurde gefunden, daß die erfindungsgemäßen peptidischen Verbindungen und ihre Salze als lösliche Moleküle Wirkung auf Zellen ausüben, die den genannten Rezeptor tragen, oder wenn sie an Oberflächen gebunden sind, künstliche Liganden für die $\alpha_v\beta_6$ - vermittelte Zellanhaftung darstellen. Vor allem wirken sie als $\alpha_v\beta_6$ Integrin-Inhibitoren, wobei sie insbesondere die Wechselwirkungen des Rezeptors mit anderen Liganden hemmen, wie z. B. die Bindung von Fibronektin. Diese Wirkung kann z.B. nach der Methode nachgewiesen werden, die von J.W. Smith et al. in J. Biol. Chem. 265, 12267-12271 (1990) beschrieben wird.

Weiter wurde gefunden, daß die neuen Substanzen bei guter Verträglichkeit sehr wertvolle pharmakologische Eigenschaften besitzen und als Arzneimittel eingesetzt werden können. Dies wird weiter unten genauer beschrieben.

Die erfindungsgemäßen peptidischen Verbindungen können ferner als Diagnostika zur Detektion und Lokalisierung von pathologischen Zuständen im epithelialen System *in vivo* und *in vitro* verwendet werden, wenn sie mit entsprechenden Markern (z.B. dem Biotinylrest) nach dem Stand der Technik ausgestattet sind.

Die Erfindung umfaßt auch Kombinationen mit mindestens einem anderen Wirkstoff und/oder Konjugate mit anderen Wirkstoffen, wie zytotoxischen Wirkstoffen sowie Konjugate mit Radiomarkern für Röntgentherapie oder PET Diagnose aber auch Fusionsproteine mit Markerproteinen wie GFP oder Antikörpern, oder therapeutischen Proteinen wie IL-2.

Besonders wirksame Verbindungen sind solche der Formel I, in denen eine Octapeptidsequenz Cyclo-(Arg-$X^1$-Asp-$X^2$-$X^3$-$X^4$-$X^5$-$X^6$), worin die Reste $X^1$, $X^2$, $X^3$, $X^4$, $X^5$ und $X^6$ die angegebenen Bedeutungen haben, durch $R^1$ aufgeweitet ist. Der Effekt der Ringaufweitung ist am Beispiel des Cyclo-(Arg-Gly-Asp-Leu-D-Asp-Ala-Leu-Arg) [EMD 271588] aufgezeigt. Als Vergleichsverbindung dient Ac-Arg-Thr-Asp-Leu-Asp-Ser-Leu-Arg-NH$_2$ [EMD 271293].

In der nachfolgenden Tabelle sind einige Peptide Cyclo-(Arg-Gly-Asp-Leu-D-Asp-Ala-Leu-Arg-$R^1$), der Abstand von $R^1$ (berechnet) und der Wert Q (IC$_{50}$[Substanz] / IC$_{50}$[EMD 271293]) bzw. -log Q angegeben.

| $R^1$ | Abstand [pm] | Q | -log Q |
|---|---|---|---|
| | | | |
| 0 | 0 | 47 | -1,672 |
| Gly | 370 | 2,1 | -0,322 |
| Abu | 617 | 0,028 | 1,553 |
| Gly-Gly | 740 | 0,05 | 1,301 |
| Aha | 870 | 0,036 | 1,444 |
| Aee | 1078 | 0,038 | 1,420 |
| Gly-Gly-Gly | 1110 | 0,03 | 1,523 |
| Abu-Abu | 1235 | 0,03 | 1,523 |
| Gly-Gly-Gly-Gly | 1480 | 0,033 | 1,481 |
| Aha-Aha | 1740 | 0,036 | 1,444 |
| Gly-Gly-Gly-Gly-Gly | 1850 | 0,046 | 1,337 |
| Gly-(Gly)$_4$-Gly | 2220 | 0,05 | 1,301 |
| | | | |

Besonders wirksame Verbindungen werden schon erhalten, wenn die Spacerlänge $R^1$ ungefähr 500 pm erreicht hat.

Besonders bevorzugt sind solche Verbindungen der Formel I, worin $R^1$ einen oder mehrere $\omega$-Aminocarbonsäurerest (e), wobei der oder die $\omega$-Aminocarbonsäurerest(e) eine Länge von 600 bis 2500 pm aufweisen, ganz besonders bevorzugt solche mit einer Spacerlänge von 600 bis 2000 pm.

Unter $\omega$-Aminocarbonsäurerest(e) versteht man irgendeine $\omega$-Aminocarbonsäure, unter denen die nachstenden Aminosäuren ausgewählt aus der Gruppe

Ala, Asn, Asp, Arg, Cys, Gln, Glu, Hcy, His, Hse, Ile, Leu, Lys, Met, Pen, Phe, Pro, Ser, Thr, Trp, Tyr, Val und $H_2N$-$(CH_2CH_2O)_m$-$(CH_2)_n$-COOH,

mit m, n    jeweils unabhängig voneinander 0, 1, 2, 3, 4, 5, 6, 7, 8, 9, 10, 11 oder 12,

mit der Maßgabe, daß m + n > 0 sind,

besonders bevorzugt sind.

Gegenstand der Erfindung sind somit bevorzugt Peptidderivate nach Anspruch 1 der Formel 1

$$Cyclo\text{-}(Arg\text{-}X^1\text{-}Asp\text{-}X^2\text{-}X^3\text{-}X^4\text{-}X^5X^6\text{-}R^1)$$

worin

| | |
|---|---|
| $X^1$ | Ser, Gly oder Thr, |
| $X^2$ | Leu, Ile, Nle, Val oder Phe, |
| $X^3$ | Asp, Glu, Lys oder Phe, |
| $X^4$ | Gly, Ala oder Ser, |
| $X^5$ | Leu, Ile, Nle, Val oder Phe, |
| $X^6$ | Arg, Har oder Lys, |
| $R^1$ | 1-10 Aminosäuren ausgewählt aus der Gruppe Ala, Asn, Asp, Arg, Cys, Gln, Glu, Hcy, His, Hse, Ile, Leu, Lys, Met, Pen, Phe, Pro, Ser, Thr, Trp, Tyr, Val und $H_2N\text{-}(CH_2CH_2O)_m\text{-}(CH_2)_n\text{-}COOH$, |
| m, n | jeweils unabhängig voneinander 0, 1, 2, 3, 4, 5, 6, 7, 8, 9, 10, 11 oder 12, |

mit der Maßgabe, daß m + n > 0 sind,
bedeuten,
wobei die D- als auch die L-Formen der optisch aktiven Aminosäurereste eingeschlossen sind,
sowie deren physiologisch unbedenklichen Salze.

Einige bevorzugte Gruppen von Verbindungen können durch die folgenden Teilformeln la bis Ih ausgedrückt werden, die der Formel I entsprechen und worin die nicht näher bezeichneten Reste die bei der Formel I angegebene Bedeutung haben, worin jedoch

in a)

| | |
|---|---|
| $X^1$ | Gly oder Thr, |

bedeutet;

in b)

| | |
|---|---|
| $X^1$ | Gly oder Thr, |
| $X^2$ | Leu, |

bedeutet;

in c)

| | |
|---|---|
| $X^1$ | Gly oder Thr, |
| $X^2$ | Leu, |
| $X^3$ | Asp oder D-Asp |

bedeutet;

in d)

| | |
|---|---|
| $X^1$ | Gly oder Thr, |
| $X^2$ | Leu, |
| $X^3$ | Asp oder D-Asp, |
| $X^4$ | Gly oder Ala, |

bedeutet;

in e)

| | |
|---|---|
| $X^1$ | Gly oder Thr, |

X²     Leu,
X³     Asp oder D-Asp,
X⁴     Gly oder Ala,
X⁵     Leu

bedeutet;

in f)

$X^1$     Gly oder Thr,
$X^2$     Leu,
$X^3$     Asp oder D-Asp,
$X^4$     Gly, Ala oder Ser,
$X^5$     Leu,
$X^6$     Arg

bedeutet;

in g)

$X^1$     Gly oder Thr,
$X^2$     Leu,
$X^3$     Asp oder D-Asp,
$X^4$     Gly, Ala oder Ser,
$X^5$     Leu,
$X^6$     Arg,
$R^1$     1-10 Aminosäuren ausgewählt aus der Gruppe Ala, Asn, Asp, Arg, Cys, Gin, Glu, Hcy, His, Hse, Ile, Leu, Lys, Met, Pen, Phe, Pro, Ser, Thr, Trp, Tyr, Val und $H_2N$-$(CH_2CH_2O)_m$-$(CH_2)_n$-COOH,
m, n     jeweils unabhängig voneinander 0, 1, 2, 3, 4, 5, 6, 7, 8, 9, 10, 11 oder 12,

mit der Maßgabe, daß m + n > 0 sind,
bedeutet;

in h)

$X^1$     Gly oder Thr,
$X^2$     Leu,
$X^3$     Asp oder D-Asp,
$X^4$     Gly, Ala oder Ser,
$X^5$     Leu,
$X^6$     Arg,
$R^1$     1-6 Aminosäuren ausgewählt aus der Gruppe Gly, β-Ala, Abu oder Aha,

bedeutet;

sowie deren Salze.

Gegenstand der Erfindung sind insbesondere peptidische Verbindungen ausgewählt aus der Gruppe

Cyclo-(Arg-Gly-Asp-Leu-Asp-Ala-Leu-Arg-Gly-Gly-Gly),
Cyclo-(Arg-Gly-Asp-Leu-Asp-Gly-Leu-Arg-Gly-Gly-Gly),
Cyclo-(Arg-Gly-Asp-Leu-D-Ala-Ala-Leu-Arg-Gly-Gly-Gly),
Cyclo-(Arg-Thr-Asp-Leu-D-Asp-Ala-Leu-Arg-Gly-Gly-Gly),
Cyclo-(Arg-Gly-Asp-Leu-D-Asp-Ala-Leu-Arg-Abu-Abu),
Cyclo-(Arg-Gly-Asp-Leu-D-Asp-A1a-Leu-Arg-Aha-Aha),
Cyclo-(Arg-Gly-Asp-Leu-D-Asp-Ala-Leu-Arg-Aha),
Cyclo-(Arg-Gly-Asp-Leu-D-Asp-Ala-Leu-Arg-Aee),
Cyclo-(Arg-Thr-Asp-Leu-D-Asp-Ala-Leu-Arg-Abu-Abu),
Cyclo-(Arg-Thr-Asp-Leu-D-Asp-Ala-Leu-Arg-β-Ala),

Cyclo-(Arg-Gly-Asp-Leu-D-Asp-Ala-Leu-Arg-β-Ala),

sowie deren physiologisch unbedenklichen Salze.

Die vor- und nachstehend aufgeführten Abkürzungen von Aminosäureresten stehen für die Reste folgender Aminosäuren:

| | |
|---|---|
| Abu | 4-Aminobuttersäure |
| Aee | $H_2N-(CH_2CH_2O)_2-CH_2COOH$ |
| Aha | 6-Aminohexansäure, 6-Aminocapronsäure |
| Aib | α-Amino-isobuttersäure |
| Ala | Alanin |
| Asn | Asparagin |
| Asp | Asparaginsäure |
| Arg | Arginin |
| Bgl | C-alpha-tert.-Butylglycin |
| Cys | Cystein |
| Dab | 2,4-Diaminobuttersäure |
| Dap | 2,3-Diaminopropionsäure |
| Gln | Glutamin |
| Glp | Pyroglutaminsäure |
| Glu | Glutaminsäure |
| Gly | Glycin |
| Har | Homoarginin |
| Hcy | Homocystein |
| His | Histidin |
| homo-Phe | homo-Phenylalanin |
| Hse | Homoserin |
| Ile | Isoleucin |
| Leu | Leucin |
| Lys | Lysin |
| Met | Methionin |
| Nal | Naphth-2-yl-alanin |
| Nle | Norleucin |
| Orn | Ornithin |
| Pen | Penicillamin |
| Phe | Phenylalanin |
| Phg | Phenylglycin |
| 4-Hal-Phe | 4-Halogen-phenylalanin |
| Pro | Prolin |
| Ser | Serin |
| Thr | Threonin |
| TIS | Triisopropylsilan |
| Trp | Tryptophan |
| Tyr | Tyrosin |
| Val | Valin. |

Ferner bedeuten nachstehend:

| | |
|---|---|
| Ac | Acetyl |
| BOC | tert.-Butoxycarbonyl |
| BSA | Bovine Serum Albumin |
| CBZ oder Z | Benzyloxycarbonyl |
| DCCI | Dicyclohexylcarbodiimid |
| DCM | Dichlormethan |
| DIEA | Diethylamin |
| DMF | Dimethylformamid |
| EDCI | N-Ethyl-N,N'-(dimethylaminopropyl)-carbodiimid |
| Et | Ethyl |

| FCA | Fluoresceincarbonsäure |
|---|---|
| FITC | Fluoresceinisothiocyanat |
| Fmoc | 9-Fluorenylmethoxycarbonyl |
| FTH | Fluoresceinthioharnstoff |
| HOBt | 1-Hydroxybenzotriazol |
| Me | Methyl |
| MBHA | 4-Methyl-benzhydrylamin |
| Mtr | 4-Methoxy-2,3,6-trimethylphenyl-sulfony |
| HONSu | N-Hydroxysuccinimid |
| OBut | tert.-Butylester |
| Oct | Octanoyl |
| OMe | Methylester |
| OEt | Ethylester |
| Pbf | 2,2,4,6,7-Pentamethyl-dihydrobenzofuran-5-sulfonyl |
| Pmc | 2,2,5,7,8-Pentamethylchroman-6-sulfonyl |
| POA | Phenoxyacetyl |
| Sal | Salicyloyl |
| TBS++ | Tris buffered Saline mit 2-wertigen Kationen |
| TBSA | TBS+BSA |
| TBTU | 2-(1H-Benzotriazol-1-yl)-1,1,3-tetramethyluronium tetrafluoroborat |
| TFA | Trifluoressigsäure |
| Trt | Trityl (Triphenylmethyl). |

Sofern die vorstehend genannten Aminosäuren in mehreren enantiomeren Formen auftreten können, so sind vor- und nachstehend, alle diese Formen und auch ihre Gemische (z. B. die DL-Formen) eingeschlossen. Ferner können die Aminosäuren mit entsprechenden an sich bekannten Schutzgruppen versehen sein.

Gegenstand der Erfindung sind auch die Hydrate und Solvate, z.B. Alkoholate, dieser Verbindungen.

In die erfindungsgemäßen Verbindungen sind auch sogenannte Prodrug-Derivate eingeschlossen, d. h. mit z. B. Alkyl- oder Acylgruppen, Zuckern oder Oligopeptiden abgewandelte Verbindungen, die im Organismus rasch zu den wirksamen erfindungsgemäßen Verbindungen gespalten werden. Hierzu gehören auch bioabbaubare Polymerderivate der erfindungsgemäßen Verbindungen, wie dies z. B. in Int. J. Pharm. 115, 61-67 (1995) beschrieben ist.

In den erfindungsgemäßen Verbindungen sind neben den Verbindungen der Formel I, die über die $\alpha$-Amino- und $\alpha$-Carboxygruppen peptidartig miteinander verknüpft sind (Kopf-Schwanz-Verknüpfung), auch solche cyclischen Verbindungen enthalten, die z.B. bei Vorliegen einer funktionellen Seitenkette, wie z.B. einer SH-Gruppe, folgendermaßen verknüpft sind

Seite-Seite z.B. S-S (Disulfid),
Kopf-Seite
Seite-Schwanz.

Ferner sind in die erfindungsgemäßen Verbindungen auch Derivate mit eingeschlossen, welche aus den eigentlichen erfindungsgemäßen Peptiden und bekannten Marker-Verbindungen bestehen, die es ermöglichen, die Peptide leicht nachzuweisen. Beispiele für solche Derivate sind radioaktiv markierte, biotinylierte oder fluoreszenzmarkierte Peptide.

Fluoreszierender Farbstoffrest bedeutet vorzugsweise 7-Acetoxycoumarin-3-yl, Fluorescein-5-(und/oder 6-)yl, 2',7'-Dichlorfluorescein-5-(und 6-)yl, Dihydrotetramethylrosamin-4-yl, Tetramethylrhodamin-5-(und/oder 6-)yl, 4,4-Difluor-5,7-dimethyl-4-bora-3a,4a-diaza-s-indacen-3-ethyl oder 4,4-Difluor-5,7-diphenyl-4-bora-3a,4a-diaza-s-indacen-3-ethyl.

Geeignete funktionalisierte fluoreszierende Farbstoffreste, die als Reagenzien zur Herstellung der erfindungsgemäßen Verbindungen der Formel I dienen können, sind z. B. beschrieben in "Handbook of Fluorescent Probes and Research Chemicals, 5th Edition, 1992-1994, by R.P. Haughland, Molecular Probes, Inc.".

Die Erfindung umfaßt nicht nur die genannten Peptide sondern auch Mischungen und Zubereitungen, welche neben diesen erfindungsgemäßen Verbindungen auch andere pharmakologische Wirkstoffe oder Adjuvantien enthalten, die die primäre pharmakologische Wirkung der erfindungsgemäßen Peptide in gewünschter Weise beinflussen können.

Die erfindungsgemäßen Verbindungen und auch die Ausgangsstoffe zu ihrer Herstellung werden im übrigen nach an sich bekannten und häufig eingesetzten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten Varianten Gebrauch machen.

Vorzugsweise können die erfindungsgemäßen Peptide mittels Festphasensynthese und nachfolgender Abspaltung und Reinigung hergestellt werden, wie dies z.B. von *Jonczyk und Meienhofer* (Peptides, Proc. 8th Am. Pept. Symp., Eds. V. Hruby und D.H.Rich, Pierce Comp. III, p. 73-77, 1983, oder Angew. Chem. 104, 1992, 375) oder gemäß Merrifield (J. Am. Chem. Soc. 94, 1972, 3102) beschrieben wurde.

Die erfindungsgemäßen Peptide können an fester Phase (manuell oder in einem Syntheseautomaten) in einer Fmoc-Strategie mit säurelabilen Seitenschutzgruppen hergestellt und mittels RP-HPLC gereinigt werden. Die Peakeinheitlichkeit kann durch RP-HPLC und die Substanzidentität mittels FAB-MS gemessen werden.

Im übrigen können die Peptide nach üblichen Methoden der Aminosäure- und Peptidsynthese hergestellt werden, wie dies z. B. aus Novabiochem - 1999 Catalog & Peptide Synthesis Handbook der Calbiochem-Novabiochem GmbH, D-65796 Bad Soden, aus zahlreichen Standardwerken und publizierten Patentanmeldungen bekannt ist.

Es können schrittweise Kupplungen und Fragmentkondensationen genutzt werden. Unterschiedliche N-terminale, C-terminale und Seitenschutzgruppen können Verwendung finden, die bevorzugt orthogonal spaltbar ausgewählt werden. Kupplungsschritte können mit unterschiedlichen Kondensationsreagenzien wie Carbodiimiden, Carbodiimidazol, solchen des Uronium-Typs wie TBTU, gemischten Anhydrid-Methoden, sowie Säurehalogenid- oder Aktivester-Methoden durchgeführt werden. Aktivierte Ester werden zweckmäßig in situ gebildet, z. B. durch Zusatz von HOBt oder N-Hydroxysuccinimid.

Die Cyclisierung eines linearen Vorläufermoleküls mit Seitenschutzgruppen ist ebenfalls mit solchen Kondensationsreaktionen durchführbar, wie z. B. in DE 43 10 643 oder in Houben-Weyl, I.c., Band 15/II, Seiten 1 bis 806 (1974) beschrieben.

Bei der Festphasenpeptidsynthese sind unterschiedliche Harze und Ankerfunktionen nutzbar. Harze können z.B. auf Polystyrol oder Polyacrylamid basieren, Ankerfunktionen wie Wang, o-Chlortrityl sind zur Herstellung von Peptidsäuren, Aminoxanthenoxy-Anker z.B. zur Herstellung von Peptidamiden nutzbar.

Biotinylierte oder fluoreszenzmarkierte Peptide / Proteine können ebenfalls nach Standardmethoden hergestellt werden. (z.B. E.A. Bayer and M. Wilchek in Methods of Biochemical Analysis Vol 26 The Use of the Avidin-Biotin Complex as a Tool in Molecular Biology; und Handbook of Fluorescent Probes and Research Chemicals, 6th Edition, 1996, by R. P. Haugland, Molecular Probes, Inc.; oder auch *WO 97/14716)*

Selbstverständlich können die erfindungsgemäßen Peptide auch durch Solvolyse, insbesondere Hydrolyse, oder durch Hydrogenolyse ihrer funktionellen Derivate freigesetzt werden. Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe oder die anstelle des H-Atoms einer Hydroxygruppe eine Hydroxyschutzgruppe tragen. Entsprechendes gilt für Carbonsäuren, die durch Substitution ihrer -CO-OH Hydroxyfunktion mittels einer Schutzgruppe, z.B. als Ester geschützt werden können.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Das In-Freiheit-Setzen der Verbindungen aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden.

Typische Schutzgruppen für N-Termini und für seitenständige Aminogruppen sind Z, BOC, Fmoc, solche für C-Termini oder die Asp- oder Glu-Seitenketten sind O-prim.-Alkyl (z.B. OMe oder OEt), O-tert.-Alkyl (z.B. OBut) oder OBenzyl. Für die Guanidinofunktion des Arg ist z.B. Z, BOC, $NO_2$, Mtr, Pmc oder Pbf geeignet. Alkoholische Funktionen können durch Benzylreste, tert.-Alkylreste oder Tritylgruppen geschützt sein.

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird z.B. zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Wie bereits erwähnt, umfassen die erfindungsgemäßen Peptide ihre physiologisch unbedenklichen Salze, welche ebenfalls nach Standardmethoden hergestellt werden können. So kann eine Base einer erfindungsgemäßen Verbindung mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der erfindungsgemäßen Verbindungen verwendet werden. Andererseits kann eine Säure der erfindungsgemäßen Verbindungen durch Umsetzung mit einer Base in eines ihrer physiologisch unbedenklichen Metall- oder Ammoniumsalze übergeführt werden. Als Salze kommen dabei insbesondere die Natrium-, Kalium-, Magnesium-, Calcium- und Ammoniumsalze in Betracht, ferner substituierte Ammoniumsalze, z. B. die Dimethyl-, Diethyl- oder Diisopropylammoniumsalze, Monoethanol-, Diethanol- oder Diisopropylammoniumsalze, Cyclohexyl-, Dicyclohexyl-ammoniumsalze, Dibenzylethylendiammoniumsalze, weiterhin z. B. Salze mit Arginin oder Lysin.

Die erfindungsgemäßen peptidischen Verbindungen können, wie bereits erwähnt, als Arzneimittelwirkstoffe in der Human- und Veterinärmedizin eingesetzt werden, insbesondere zur Prophylaxe und/oder Therapie von Erkrankungen bei denen epitheliale Zellen beteiligt sind. Besonders hervorzuheben sind hierbei Erkrankungen oder Entzündungen oder Wundheilungsprozesse der Haut, der Atemwegorgane und des Magen- und Darmbereichs, so zum Beispiel Apoplexie, Angina pectoris, Tumorerkrankungen, osteolytischen Krankheiten wie Osteoporose, pathologisch angiogenen Krankheiten wie z. B. Entzündungen, Fibrosen, insbesondere Lungenfibrose, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, Restenose nach Angioplastie, bei akutem Nierenversagen, Nierenentzündung, mikrobiellen Infekten und Multipler Sklerose.

Gegenstand der Erfindung sind demgemäß peptidische Verbindungen der oben und unten sowie in den Ansprüchen definierten Formeln einschließlich ihrer physiologisch unbedenklichen Salze als Arzneimittel, Diagnostika oder Reagenzien.

Gegenstand der Erfindung sind insbesondere entsprechende Arzneimittel als Inhibitoren zur Bekämpfung von Erkrankungen, die mittelbar oder unmittelbar auf einer Expression des $\alpha_v\beta_6$-Integrinrezeptors beruhen, insbesondere also bei pathologisch angiogenen Erkrankungen, Thrombosen, Herzinfarkt, koronaren Herzerkrankungen, Arteriosklerose, Tumoren, Osteoporose, Entzündungen, Infektionen sowie zur Beeinflussung von Wundheilungsprozessen.

Gegenstand sind auch entsprechende pharmazeutische Zubereitungen, welche mindestens ein Arzneimittel der Formel I sowie gegebenenfalls Träger- und/oder Hilfsstoffe enthalten.

Ferner ist Gegenstand der Erfindung die Verwendung der peptidischen Verbindungen und/oder ihre physiologisch unbedenklichen Salze gemäß der Ansprüche und der Beschreibung zur Herstellung eines Arzneimittels zur Bekämpfung von Erkrankungen, die mittelbar oder unmittelbar auf einer Expression des $\alpha_v\beta_6$-Integrinrezeptors beruhen, insbesondere also bei pathologisch angiogenen Erkrankungen, Thrombosen, Herzinfarkt, koronaren Herzerkrankungen, Arteriosklerose, Tumoren, Osteoporose, Entzündungen, Infektionen sowie zur Beeinflussung von Wundheilungsprozessen.Die erfindungsgemäßen Arzneimittel bzw. sie enthaltende pharmazeutische Zubereitungen können in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.

Für die Applikation als Inhalationsspray können Sprays verwendet werden, die den Wirkstoff entweder gelöst oder suspendiert in einem Treibgas oder Treibgasgemisch (z. B. $CO_2$ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die erfindungsgemäßen Substanzen können in der Regel in Analogie zu anderen bekannten, im Handel befindlichen Peptiden (z.B. beschrieben in der US-A-4 472 305) verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 20 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die neuen Verbindungen der Formel I in der analytischen Biologie und Molekularbiologie verwendet werden.

Die neuen Verbindungen der Formel I, wobei X einen über eine -CONH-, -COO-, -NH-C(=S)-NH-, -NH-C(=O)-NH- , -SO_2NH- oder -NHCO- Bindung verknüpften fluoreszierenden Farbstoffrest bedeutet, können als diagnostische Marker in der FACS (Fluorescence Activated Cell Sorter)-Technik und Fluoreszenz-Mikroskopie verwendet werden.

Der Einsatz von markierten Verbindungen in der Fluoreszenz-Mikroskopie ist z. B. beschrieben von Y.-L. Wang und D. L. Taylor in "Fluorescence Microscopy of Living Cells in Culture, Part A + B, Academic Press, Inc. 1989".

Die neuen erfindungsgemäßen Verbindungen können auch als Integrinliganden zur Herstellung von Säulen für die Affinitätschromatographie zur Reindarstellung von Integrinen verwendet werden. Der Komplex aus einem Avidinderivatisierten Trägermaterial, z.B. Sepharose und den neuen Verbindungen wird nach an sich bekannten Methoden (z.B. E.A. Bayer and M. Wilchek in Methods of Biochemical Analysis Vol 26 The Use of the Avidin-Biotin Complex as a Tool in Molecular Biology) gebildet. Als polymere Trägermaterialien eignen sich dabei die an sich in der Peptidchemie bekannten polymeren festen Phasen mit vorzugsweise hydrophilen Eigenschaften, beispielsweise quervernetzte Polyzucker wie Cellulose, Sepharose oder Sephadex$^R$, Acrylamide, Polymer auf Polyethylenglykolbasis oder Tentakelpolymere$^R$.

Die Erfindung umfaßt schließlich auch rekombinante DNA-Sequenzen, welche Abschnitte enthalten, die für Peptidbereiche codieren, die die erfindungsgemäßen peptidischen Strukturmotive aufweisen.

Solche DNA kann durch Partikel auf Zellen übertragen werden, wie in Ch. Andree et al. Proc.Natl.Acad.Sci. 91, 12188-12192 (1994) beschrieben ist, oder der Transfer auf Zellen kann durch andere Hilfsmittel, wie Liposomen, gesteigert werden (A.I. Aronsohn and J.A. Hughes J. Drug Targeting, 5, 163-169 (1997)).

Der Transfer einer solchen DNA könnte demnach in Hefen, mittels Bacculo-Viren oder in Säugerzellen für die Produktion der peptidischen Substanzen dieser Erfindung benutzt werden.

Wird ein tierischer oder menschlicher Organismus mit solch einer rekombinanten DNA infiziert, dann können die durch die infizierten Zellen letztlich selbst gebildeten erfindungsgemäßen Peptide unmittelbar an den $\alpha_v\beta_6$ —Integrinrezeptor, beispielsweise von Tumorzellen binden und ihn blockieren.

Entsprechende rekombinante DNA, die durch bekannte und übliche Techniken bereitgestellt werden kann, kann beispielsweise aber auch in Form von Virus-DNA vorliegen, welche Abschnitte enthält, die für das Virus-Hüllprotein codieren. Durch Infektion eines Wirtsorganismus mit derartigen rekombinanten, vorzugsweise nicht pathogenen Viren, können Wirtszellen, die das Integrin $\alpha_v\beta_6$ exprimieren, bevorzugt angegriffen werden (Targetierung).

Geeignete Viren sind beispielsweise Adenovirenarten, die mehrfach schon als Vektoren für fremde Gene in Säugerzellen benutzt wurden. Eine Anzahl von Eigenschaften machen sie zu guten Kandidaten für Gentherapie, wie S.J. Watkins et al. Gene Therapy 4, 1004-1012 (1997) zu entnehmen ist (siehe auch J. Engelhardt et al. Hum. Gene Ther. 4, 759-769(1993)).

Wie in A. Fasbender et al. J.Clin.Invest. 102, 184-193 (1998) zu finden ist, ist gemeinsames Problem bei Gentherapie durch virale und nichtvirale Vektoren die limitierte Effizienz des Gentransfers. Mit der oben beschriebenen zusätzlichen Ligandensequenz für $\alpha_v\beta_6$ Integrin im Hüllprotein der Adenoviren kann eine Verbesserung des Transfers z.B. von Cystic Fibrosis Transmembrane Conductance Regulator (CFTR) cDNA erreicht werden.

Ähnlich wie in der Arbeit von T. Tanaka et al. Cancer Research 58, 3362-3369 (1998) kann statt der DNA für Angiostatin auch die DNA für die Sequenzen dieser Erfindung für Zelltransfektionen mittels retroviraler oder adenoviraler Vektoren genutzt werden.

Die erfindungsgemäßen Peptide können auch innerhalb eines Liposomekomplexes aus Lipid/Peptid/DNA für eine Transfektion von Zellkulturen zusammen mit einem Liposomen-Komplex bestehend aus Lipid/ DNA (ohne Peptid) für den Einsatz in der Gentherapie am Menschen eingesetzt werden.Die Herstellung eines Liposomenkomplexes aus Lipid/DNA/Peptid ist beispielsweise bei Hart S.L, et al 1998: Lipid-Mediated Enhancement of Transfection by a Non-Viral Integrin-Targeting Vector. Human Gene Therapy 9, 575-585, beschrieben.

Ein Liposomenkomplex aus Lipid/Pepid/DNA ist beispielsweise aus folgenden Stammlösungen herstellbar:1µg/µl Lipofectin (äquimolare Mischung aus DOTMA (= N-[1-(2,3-dioleyloxy) propyl]-N,N,N-trimethylammonium Chlorid) und DOPE (Dioleyl Phosphatidylethanolamin)), 10 µg/ml Plasmid DNA und 100 µg/ml Peptid. Sowohl DNA als auch Peptid werden dazu in Zellkulturmedium gelöst.

Der Liposomenkomplex wird durch Mischen der drei Komponenten in einem bestimmten Gewichtsverhältnis (Lipid: DNA: Peptid, z.B. 0,75 : 1 : 4) hergestellt. Liposomen DNA-Komplexe für eine Gentherapie am Menschen sind bereits beschrieben worden (Caplen N.J., et al 1995: Liposome-mediated CFTR gene transfer to the nasal epithelium of patients with cystic fibrosis Nature Medicine 1, 39-46).

Gegenstand der Erfindung ist somit auch die Verwendung entsprechend modifizierter rekombinater DNA von Gen-freisetzenden Systemen, insbesondere Virus-DNA, zur Bekämpfung von Krankheiten welche mittelbar oder unmittelbar auf einer Expression von $\alpha_v\beta_6$ —Integrinrezeptoren beruhen, insbesondere also bei pathologisch angiogenen Erkrankungen, Thrombosen, Herzinfarkt, koronaren Herzerkrankungen, Arteriosklerose, Tumoren, Osteoporose, Entzündungen, Infektionen sowie zur Beeinflussung von Wundheilungsprozessen.

Vor- und nachstehend sind alle Temperaturen in °C angegeben.

Die HPLC-Analysen (Retentionszeit Rt) erfolgten in den folgenden Systemen:

Säule 5 µm LichroSpher 60 RP-Select B (250-4), mit einem 50-minütigen Gradienten von 0 bis 80% 2-Propanol in Wasser / 0,3% Trifluoressigsäure, bei 1 ml/min Fluss und Detektion bei 215 nm.

Massenspektrometrie (MS): EI (Elektronenstoß-Ionisation) M+
FAB (Fast Atom Bombardment) (M+H)+

Beispiel 1

*Herstellung und Aufreinigung erfindungsgemäßer Peptide:*

Prinzipiell erfolgte die Herstellung und Aufreinigung mittels Fmoc-Strategie unter Protektion säurelabiler Seitenketten auf särelabilen Harzen unter Benutzung eines kommerziell erhältlichen "continuous flow" Peptidsynthesizers entsprechend den Angaben von Haubner et al. (J. Am. Chem. Soc. 118, 1996, 17703).

Cyclo-(Arg-Thr-Asp-Leu-D-Asp-Ala-Leu-Arg-Abu-Abu) [EMD 272914]

2,7 g Fmoc-Abu-OH (Bachem B-1910) wurde in 150 ml Methylenchlorid suspendiert und mit 10 ml DMF klar gelöst. Es wurden 5,6 ml Diisopropylethylamin zugegeben, die Lösung auf 12,0 g o-Chlortrityl-Chlorid-Polystyrolharz (1,14 mmol/g, Bachem D-196512) gegossen und der Ansatz bei Raumtemperatur geschüttelt. Nach 5 Stunden wurde das Harz abgesaugt, mit je 300 ml DCM/MeOH/DIEA = 17/2/1, DCM, DMF,DCM und MeOH gewaschen. Nach Entfernung der Lösungsmittel wurden 14,55 g Fmoc-Aminosäure-Harz erhalten. Eine dreifache Fmoc-Bestimmung ergab im Mittelwert eine Beladung von 541 µmol/g Fmoc-Abu-O-oClTrt-Harz.

0,7 g Fmoc-Abu-O-oClTrt-Polystyrol-Harz wurden nacheinander in einer Doppelkupplungstechnik 2 x mit je 0,30 g TBTU, 0,315 ml Ethyldiisopropylamin und Fmoc-Aminosäure in 4,1 ml DMF in einem kommerziellen Synthesegerät und einer typischen Prozedur (Gerät und Handbuch Milligen 9050 Pepsynthesizer™, 1987), für jeweils 30 Minuten, einem Kupplungsschritt unterworfen. Waschschritte erfolgten in DMF für 10 Minuten, Abspaltungsschritte in Piperidin/DMF (1:4 vol) für 5 Minuten, N-terminale Acetylierungen (Capping) wurden mit Essigsäureanhydrid / Pyridin / DMF (2:3:15 vol) für 15 Minuten durchgeführt.

Es kamen die Aminosäuren Fmoc-Arg(Pmc), danach Fmoc-Leu, danach Fmoc-Ala, danach Fmoc-D-Asp(OBut), danach Fmoc-Leu, danach Fmoc-Asp(OBut), danach Fmoc-Thr(But) danach Fmoc-Arg(Pmc) und schließlich Fmoc-Abu zum Einsatz. Nach Abspalten der Fmoc-Schutzgruppe vom Fmoc-Abu-Arg(Pmc)-Thr(But)-Asp(OBut)-Leu-D-Asp(OBut)-Ala-Leu-Arg(Pmc)-Abu-O-oClTrt-Polystyrolharz wurde mit DMF und Isopropanol gewaschen und nach Trocknung am Vakuum bei Raumtemperatur wurden 0,9 g Abu-Arg(Pmc)-Thr(But)-Asp(OBut)-Leu-D-Asp(OBut)-Ala-Leu-Arg(Pmc)-Abu-O-oClTrt-Polystyrolharz erhalten.

Durch Behandlung dieses Peptidylharzes mit 20 ml Trifluorethanol/Dichlormethan/Essigsäure (2:6:2 vol) für 2 Stunden bei Raumtemperatur, Filtration, Einengen im Vakuum und Verreiben mit Diethylether wurde 0,19 g des seitenkettengeschützten Peptides Abu-Arg(Pmc)-Thr(But)-Asp(OBut)-Leu-D-Asp(OBut)-Ala-Leu-Arg(Pmc)-Abu-OH gewonnen.

Durch Zutropfen einer Lösung dieses Produktes in DMF (100 mg Peptid /15 ml DMF) in eine gerührte Lösung von TBTU/HOBt/DIPEA (10:10:11 Äquivalente) in 50 ml DMF /100 mg Peptid innerhalb von 30 Minuten und weiteres Rühren für 1 Stunde konnte die Cyclisierung erreicht werden. Nach Einengen und Ausfällen mit Wasser wurden 0,15 g rohes Cyclo-(Arg(Pmc)-Thr(But)-Asp(OBut)-Leu-D-Asp(OBut)-Ala-Leu-Arg(Pmc)-Abu-Abu) gewonnen. Nach Behandlung mit Trifluoressigsäure/Wasser/TIS (94:3:3 vol) für 2 Stunden bei Raumtemperatur, Einengen im Vakuum und Verreiben mit Diethylether fiel ein Niederschlag von 85 mg Cyclo-(Arg-Thr-Asp-Leu-D-Asp-Ala-Leu-Arg-Abu-Abu) an.

Eine Reinigung des Produktes erfolgte per RP-HPLC auf Lichrosorb RP 18 (250 - 25, 7 μm, Merck KGaA) in 0,3 % TFA mit einem Gradienten von 4 % auf 24 % 2-Propanol in einer Stunde bei 10 ml/min und Beurteilung des Eluats mittels UV-Durchflussphotometer bei 215 und 254 nm. Es wurden 51 mg Produkt erhalten, FAB 1067; Rt 19,0.

Analog wurden die folgenden Produkte hergestellt:

| Code (EMD) | Sequenz | MW (g/mol) | FAB | Rt |
|---|---|---|---|---|
| 271312 | cyclo-(RTDLDSLR) | 957,1 | 958 | 15,10 |
| 271578 | cyclo-(RTDLdSLR) | 957,1 | 957 | 17,51 |
| 271579 | cyclo-(RTDLdALR) | 941,1 | 941 | 16,64 |
| 271586 | cyclo-(RGDLDSLR) | 913,0 | 913 | 17,95 |
| 271587 | cyclo-(RGDLdSLR) | 913,0 | 913 | 17,24 |
| 271588 | cyclo-(RGDLdALR) | 897,0 | 897 | 19,60 |
| 271589 | cyclo-(RGDLdGLR) | 882,9 | 883 | 16,68 |
| 271590 | cyclo-(RGDLd-β-Ala-LR) | 897,0 | 897 | 15,47 |
| 271591 | cyclo-(RGDLdALRG) | 954,1 | 954 | 16,79 |
| 271592 | cyclo-(RGDLdALRGG) | 1011,1 | 1011 | 17,83 |
| 271593 | cyclo-(RGDLdALRGGG) | 1068,2 | 1068 | 17,79 |
| 271594 | cyclo-(RGDLdALRGGGGGG) | 1239,3 | 1239 | 17,30 |
| 272914 | cyclo-(RGDLdALR-Abu-Abu) | 1067,2 | 1067 | 19,00 |
| 272966 | cyclo-(RTDLdALRGGG) | 1112,2 | 1113 | 17,14 |
| 272967 | cyclo-(RTDLdGLRGGG) | 1098,2 | 1099 | 19,00 |
| 272968 | cyclo-(RTDLDALRGGG) | 1112,2 | 1113 | 17,39 |
| 272969 | cyclo-(RTDLDGLRGGG) | 1098,2 | 1099 | 16,88 |
| 272970 | cyclo-(RGDLDALRG) | 954,1 | 955 | 17,32 |
| 272971 | cyclo-(RaDLdALRGGG) | 1082,2 | 1083 | 18,26 |
| 272972 | cyclo-(RGDLaALRGGG) | 1082,2 | 1083 | 18,23 |
| 272958 | cyclo-(RGDLdALR-Aha-Aha) | 1123,3 | 1123 | 19,38 |
| 272959 | cyclo-(RGDLdALR-Aha) | 1010,2 | 1010 | 20,63 |
| 272960 | cyclo-(RGDLdALR-Aee) | 1042,2 | 1042 | 18,92 |
| 272961 | cyclo-(RGDLdALRGGGG) | 1125,2 | 1125 | 17,56 |
| 272962 | cyclo-(RGDLdALRGGGGG) | 1182,3 | 1182 | 17,52 |
| 272963 | cyclo-(RGDLdGLRGGG) | 1054,1 | 1054 | 15,59 |
| 272964 | cyclo-(RGDLDALRGGG) | 1068,2 | 1069 | 16,84 |
| 272965 | cyclo-(RGDLDGLRGGG) | 1054,1 | 1055 | 16,03 |
| 273028 | cyclo-(RTDLdALR-Aha) | 1072,2 | n.d. | 19,30 |
| 273033 | cyclo-(RTDLdALR-Abu) | 1044,2 | n.d. | 16,72 |
| 273035 | cyclo-(RGDLdALR-Abu) | 1000,1 | n.d. | 16,96 |
| 273038 | cyclo-(RTDLdALR-Aha-Aha) | 1185,4 | n.d. | 20,46 |

(fortgesetzt)

| Code (EMD) | Sequenz | MW (g/mol) | FAB | Rt |
|---|---|---|---|---|
| 273040 | cyclo-(RTDLdALR-Abu-Abu) x 2 TFA | 1111,3 | n.d. | 18,75 |
| 304219 | cyclo-(RTDLdALR-βAla) | 1012,1 | 1012 | 19,4 |
| 304218 | cyclo-(RGDLdALR-βAla) | 968,1 | 968 | 18,7 |
| 329400 | cyclo-(RGDLdALR) | 811,9 | 812 | 21,15 |
| 329412 | cyclo-(RTDLdALR-Abu-Abu) | 1111,3 | 1112 | 20,31 |
| 329402 | cyclo-(RGDLdALAGGG) | 983,1 | 984 | 20,48 |
| 329399 | cyclo-(NMeArg-GDLaALRGGG) | 1038,2 | 1039 | 19,66 |
| 329398 | cyclo-(R-NMeGly-DLaALRGGG) | 1038,2 | 1039 | 19,76 |
| 326397 | cyclo-(RGD-NMeLeu-aALRGGG) | 1038,2 | 1039 | 20,22 |
| 329396 | cyclo-(RGDL-[D-NMeAla]-ALRGGG) | 1038,2 | 1039 | 20,55 |
| 329395 | cyclo-(RGDLa-NMeAla-LRGGG) | 1038,2 | 1039 | 21,35 |
| 329394 | cyclo-(RGDLaA-NMeLeu-RGGG) | 1038,2 | 1039 | 18,87 |
| 329393 | cyclo-(RGDLaAL-NMeArg-GGG) | 1038,2 | 1039 | 20,64 |
| | cyclo-(RGDLdAAR) | | | |
| | cyclo-(RGDLdAARGGG) | | | |

Nomenklatur für Aminosäuren nach Eur.J.Biochem. 138, 9-37 (1984) kleiner Buchstabe = D-Aminosäure n.d. = nicht gemessen

Die Verbindungen 271312, 271578, 271579, 271586-271591, 272970, 272971, 329393-329400 sind Keine erfindungsgemäßen Verbindungen, sondern Vergleichsverbindungen.

**Beispiel 2:**

$\alpha_v\beta_6$ / Fibronektin Rezeptorbindungstest:

Die hergestellten erfindungsgemäßen Peptide wurden in Lösung zusammen mit kompetetiv wirkenden Fibronektin an den immobiliserten $\alpha_v\beta_6$ Rezeptor gebunden und der Q-Wert als Maß für die Selektivität der Bindung des zu testenden Peptids an $\alpha_v\beta_6$ ermittelt. Der Q-Wert berechnet sich dabei aus dem Quotienten der $IC_{50}$-Werte von Testpeptid und einem Standard. Als Standard diente das lineare Ac-RTDLDSLR-NH$_2$ (Code EMD 271293) (Lit./Patent vgl Pytela et al. Science 231, 1559, (1986)). Der Bindungstest wurde im einzelnen wie folgt durchgeführt:

Die Immobilisierung von löslichem $\alpha_v\beta_6$ Rezeptor auf Microtiterplatten erfolgte durch Verdünnung der Proteinlösung in TBS++ und anschliessender Inkubation über Nacht bei 4°C (100 µl/Vertiefung). Unspezifische Bindungsstellen wurden durch Inkubation (2 h, 37°C) mit 3% (w/v) BSA in TBS++ (200 µl/Vertiefung) blockiert. Überschüssiges BSA wurde durch dreimaliges Waschen mit TBSA++ entfernt. Peptide wurden seriell (1:10) in TBSA++ verdünnt und zusammen mit biotinyliertem Fibronektin (2 µg/ ml) mit dem immobilisierten Integrin inkubiert (50 µl Peptid + 50 µl Ligand pro Vertiefung; 2 h; 37°C). Nicht gebundenes Fibronektin und Peptide wurden durch dreimaliges Waschen mit TBSA++ entfernt. Die Detektion des gebundenen Fibronektin erfolgte durch Inkubation (1 h; 37 °C) mit einem alkalische-Phosphatase-gekoppelten anti-Biotin-Antikörper (Biorad) (1:20000 in TBSA++; 100 µl/Vertiefung). Nach dreimaligem Waschen mit TBSA++ erfolgte die kolorimetrische Detektion durch Inkubation (10-15 min; 25°C, im Dunkeln) mit Substratlösung (5 mg Nitrophenylphosphat, 1 ml Ethanolamin, 4 ml H$_2$O; 100 µl/Vertiefung). Die Enzymreaktion wurde durch Zugabe von 0,4 M NaOH (100 µl/Vertiefung) gestoppt. Die Farbintensität wurde bei 405 nm im ELISA-Meßgerät bestimmt und gegen den Nullwert abgeglichen. Als Nullwert dienten Vertiefungen, die nicht mit Rezeptor beschichtet waren. Als Standard wurde Ac-RTDLDSLR-NH$_2$ eingesetzt. Die $IC_{50}$-Werte für die getesteten Peptide wurden aus einer Graphik abgelesen und daraus zusammen mit dem $IC_{50}$-Wert des Standardpeptids der Q-Wert des erfindungsgemäßen Peptids ermittelt.

# Q-Wert = $IC_{50}$ Testpeptid / $IC_{50}$ Standard

Q-Werte aus Versuchswiederholungen wurden gemittelt.

Die Ergebnisse des beschriebenen Tests sind in folgender Tabelle 1 zusammengefaßt:

Tabelle 1

| Ergebnisse des $\alpha_v\beta_6$ / Fibronektin Rezeptorbindungstests | | |
|---|---|---|
| Code (EMD) | Sequenz | Q-Wert = $IC_{50}$ Testpeptid / $IC_{50}$ EMD 271293 |
| | | |
| 271293 | Ac-RTDLDSLR-NH$_2$ | 1,00 (=75 nM) |
| 271586 | cyclo-(RGDLDSLR) | 26 |
| 271587 | cyclo-(RGDLdSLR) | 88 |
| 271588 | cyclo-(RGDLdALR) | 47 |
| 271589 | cyclo-(RGDLdGLR) | 19 |
| 272970 | cyclo-(RGDLDALRG) | 6,7 |
| 272964 | cyclo-(RGDLDALRGGG) | 0,037 |
| 272965 | cyclo-(RGDLDGLRGGG) | 0,16 |
| 272972 | cyclo-(RGDLaALRGGG) | 0,05 |
| 271593 | cyclo-(RGDLdALRGGG) | 0,03 |
| 272963 | cyclo-(RGDLdGLRGGG) | 0,084 |
| 271590 | cyclo-(RGDLd-βAla-LR) | 233 |
| 271591 | cyclo-(RGDLdALRG) | 2,1 |
| 271592 | cyclo-(RGDLdALRGG) | 0,05 |
| 271594 | cyclo-(RGDLdALRGGGGGG) | 0,05 |
| 272914 | cyclo-(RGDLdALR-Abu-Abu) | 0,03 |
| 272958 | cyclo-(RGDLdALR-Aha-Aha) | 0,036 |
| 272959 | cyclo-(RGDLdALR-Aha) | 0,036 |
| 272960 | cyclo-(RGDLdALR-Aee) | 0,038 |
| 272961 | cyclo-(RGDLdALRGGGG) | 0,033 |
| 272962 | cyclo-(RGDLdALRGGGGG) | 0,046 |
| 273035 | cyclo-(RGDLdALR-Abu) | 0,028 |
| 271312 | cyclo-(RTDLDSLR) | 8 |
| 271578 | cyclo-(RTDLdSLR) | 104 |
| 271579 | cyclo-(RTDLdALR) | 12 |
| 272966 | cyclo-(RTDLdALR-GGG) | 0,05 |
| 272967 | cyclo-(RTDLdGLR-GGG) | 0,15 |
| 272968 | cyclo-(RTDLDALR-GGG) | 0,14 |
| 272969 | cyclo-(RTDLDGLR-GGG) | 0,18 |
| 273028 | cyclo-(RTDLdALR-Aha) | 0,015 |
| 273033 | cyclo-(RTDLdALR-Abu) | 0,043 |

(fortgesetzt)

| Ergebnisse des $\alpha_v\beta_6$ / Fibronektin Rezeptorbindungstests | | |
|---|---|---|
| Code (EMD) | Sequenz | Q-Wert = $IC_{50}$ Testpeptid / $IC_{50}$ EMD 271293 |
| 273038 | cyclo-(RTDLdALR-Aha-Aha) | 0,015 |
| 273040 | cyclo-(RTDLdALR-Abu-Abu) | 0,014 |
| 272971 | cyclo-(RaDLdALR-GGG) | 8,2 |
| | | |

Die nachfolgenden Beispiele betreffen pharmazeutische Zubereitungen:

**Beispiel A: Injektionsgläser**

Eine Lösung von 100 g Cyclo-(Arg-Thr-Asp-Leu-D-Asp-Ala-Leu-Arg-Abu-Abu) und 5 g Dinatriumhydrogen-phosphat wird in 3 l zweifach destilliertem Wasser mit 2 n Salzsäure auf pH 6,5 eingestellt, steril filtriert, in Injektionsglä ser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g Cyclo-(Arg-Thr-Asp-Leu-D-Asp-Ala-Leu-Arg-Abu-Abu) mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g Cyclo-(Arg-Thr-Asp-Leu-D-Asp-Ala-Leu-Arg-Abu-Abu), 9,38 g $NaH_2PO_4 \cdot 2\,H_2O$, 28,48 g $Na_2HPO_4 \cdot 12\,H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg Cyclo-(Arg-Thr-Asp-Leu-D-Asp-Ala-Leu-Arg-Abu-Abu) mit 99,5 g Vaseline unter aseptischen Bedingungen.

**Beispiel E: Tabletten**

Ein Gemisch von 1 kg Cyclo-(Arg-Thr-Asp-Leu-D-Asp-Ala-Leu-Arg-Abu-Abu), 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Cyclo-(Arg-Thr-Asp-Leu-D-Asp-Ala-Leu-Arg-Abu-Abu) werden in üblicher Weise in Hartgelatinekapseln gefüllt, so daß jede Kapsel 20 mg des Wirkstoffs enthält.

**Beispiel H: Ampullen**

Eine Lösung von 1 kg Cyclo-(Arg-Thr-Asp-Leu-D-Asp-Ala-Leu-Arg-Abu-Abu) in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Beispiel I: Inhalations-Spray**

Man löst 14 g Cyclo-(Arg-Thr-Asp-Leu-D-Asp-Ala-Leu-Arg-Abu-Abu) in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

**Patentansprüche**

1. Peptidderivate der Formel I

$$\text{Cyclo-(Arg-}X^1\text{-Asp-}X^2\text{-}X^3\text{-}X^4\text{-}X^5\text{-}X^6\text{-}R^1) \qquad I$$

worin

$X^1$ Ser, Gly oder Thr,
$X^2$ Leu, Ile, Nle, Val oder Phe,
$X^3$ Asp, Glu, Lys oder Phe,
$X^4$ Gly, Ala oder Ser,
$X^5$ Leu, Ile, Nle, Val oder Phe,
$X^6$ Arg, Har oder Lys,
$R^1$ einen oder mehrere ω-Aminocarbonsäurerest(e), wobei der oder die ω-Aminocarbonsäurerest(e) eine Länge von 500 bis 2500 pm aufweisen,

bedeuten,
wobei
die D- als auch die L-Formen der optisch aktiven Aminosäurereste eingeschlossen sind,
sowie deren physiologisch unbedenklichen Salze und Solvate.

2. Peptidderivate nach Anspruch 1 der Formel I

$$\text{Cyclo-(Arg-}X^1\text{-Asp-}X^2\text{-}X^3\text{-}X^4\text{-}X^5\text{-}X^6\text{-}R^1) \qquad I$$

worin

$X^1$ Ser, Gly oder Thr,
$X^2$ Leu, Ile, Nle, Val oder Phe,
$X^3$ Asp, Glu, Lys oder Phe,
$X^4$ Gly, Ala oder Ser,
$X^5$ Leu, Ile, Nle, Val oder Phe,
$X^6$ Arg, Har oder Lys,
$R^1$ 1-10 Aminosäuren ausgewählt aus der Gruppe Ala, Asn, Asp, Arg, Cys, Gln, Glu, Hcy, His, Hse, Ile, Leu, Lys, Met, Pen, Phe, Pro, Ser, Thr, Trp, Tyr, Val und $H_2N\text{-}(CH_2CH_2O)_m\text{-}(CH_2)_n\text{-}COOH$,
m, n jeweils unabhängig voneinander 0, 1, 2, 3, 4, 5, 6, 7, 8, 9, 10, 11 oder 12,

mit der Maßgabe, daß m + n > 0 sind,
bedeuten,
wobei
die D- als auch die L-Formen der optisch aktiven Aminosäurereste eingeschlossen sind,
sowie deren physiologisch unbedenklichen Salze und Solvate.

3. Peptidische Verbindungen nach Anspruch 1 oder 2 ausgewählt aus der Gruppe

Cyclo-(Arg-Gly-Asp-Leu-Asp-Ala-Leu-Arg-Gly-Gly-Gly),
Cyclo-(Arg-Gly-Asp-Leu-Asp-Gly-Leu-Arg-Gly-Gly-Gly),
Cyclo-(Arg-Gly-Asp-Leu-D-Ala-Ala-Leu-Arg-Gly-Gly-Gly),
Cyclo-(Arg-Thr-Asp-Leu-D-Asp-Ala-Leu-Arg-Gly-Gly-Gly),
Cyclo-(Arg-Gly-Asp-Leu-D-Asp-Ala-Leu-Arg-Abu-Abu),

Cyclo-(Arg-Gly-Asp-Leu-D-Asp-Ala-Leu-Arg-Aha-Aha),
Cyclo-(Arg-Gly-Asp-Leu-D-Asp-Ala-Leu-Arg-Aha),
Cyclo-(Arg-Gly-Asp-Leu-D-Asp-Ala-Leu-Arg-Aee),
Cyclo-(Arg-Thr-Asp-Leu-D-Asp-Ala-Leu-Arg-Abu-Abu),
Cyclo-(Arg-Thr-Asp-Leu-D-Asp-Ala-Leu-Arg-$\beta$-Ala),
Cyclo-(Arg-Gly-Asp-Leu-D-Asp-Ala-Leu-Arg-$\beta$-Ala),

sowie deren physiologisch unbedenklichen Salze und Solvate.

4. Peptidische Verbindungen der Formel I gemäß den Ansprüchen 1-2 und die Verbindungen gemäß Anspruch 3 sowie deren physiologisch unbedenklichen Salze und Solvate als Arzneimittel.

5. Arzneimittel nach Anspruch 4 als Inhibitor zur Bekämpfung von Erkrankungen, die auf einer Expression und pathologischen Funktion von $\alpha_v\beta_6$ Integrinrezeptoren beruhen.

6. Arzneimittel nach Anspruch 5 zur Bekämpfung von Thrombosen, Herzinfarkt, koronaren Herzerkrankungen, Arteriosklerose, Tumoren, Osteoporose, Fibrosen, Entzündungen, Infektionen, Psoriasis sowie zur Beeinflussung von Wundheilungsprozessen.

7. Pharmazeutische Zubereitung, enthaltend mindestens ein Arzneimittel gemäß einem der Ansprüche 5 bis 6 sowie gegebenenfalls Träger- und/oder Hilfsstoffe und gegebenenfalls andere Wirkstoffe.

8. Verwendung von peptidischen Verbindungen gemäß der Ansprüche 1 bis 3 und/oder ihre physiologisch unbedenklichen Salze zur Herstellung eines Arzneimittels zur Bekämpfung von Erkrankungen, die auf einer Expression und pathologischen Funktion von $\alpha_v\beta_6$ Integrinrezeptoren beruhen.

9. Verwendung nach Anspruch 8 zur Herstellung eines Arzneimittels zur Bekämpfung von Thrombosen, Herzinfarkt, koronaren Herzerkrankungen, Arteriosklerose, Tumoren, Osteoporose, Fibrosen, Entzündungen, Infektionen, Psoriasis sowie zur Beeinflussung von Wundheilungsprozessen.

**Claims**

1. Peptide derivatives of the formula I

$$\text{cyclo(Arg-X}^1\text{-Asp-X}^2\text{-X}^3\text{-X}^4\text{-X}^5\text{-X}^6\text{-R}^1) \qquad \text{I}$$

in which

$X^1$ denotes Ser, Gly or Thr,
$X^2$ denotes Leu, Ile, Nle, Val or Phe,
$X^3$ denotes Asp, Glu, Lys or Phe,
$X^4$ denotes Gly, Ala or Ser,
$X^5$ denotes Leu, Ile, Nle, Val or Phe,
$X^6$ denotes Arg, Har or Lys,
$R^1$ denotes one or more $\omega$-aminocarboxylic acid radical(s), where the $\omega$-aminocarboxylic acid radical(s) have a length of 500 to 2500 pm,

where the D and L forms of the optically active amino acid radicals are included,
and physiologically acceptable salts and solvates thereof.

2. Peptide derivatives according to Claim 1 of the formula I

$$\text{cyclo(Arg-X}^1\text{-Asp-X}^2\text{-X}^3\text{-X}^4\text{-X}^5\text{-X}^6\text{-R}^1) \qquad \text{I}$$

in which

$X^1$ denotes Ser, Gly or Thr,

$X^2$ denotes Leu, Ile, Nle, Val or Phe,

$X^3$ denotes Asp, Glu, Lys or Phe,

$X^4$ denotes Gly, Ala or Ser,

$X^5$ denotes Leu, Ile, Nle, Val or Phe,

$X^6$ denotes Arg, Har or Lys,

$R^1$ denotes 1-10 amino acids selected from the group Ala, Asn, Asp, Arg, Cys, Gln, Glu, Hcy, His, Hse, Ile, Leu, Lys, Met, Pen, Phe, Pro, Ser, Thr, Trp, Tyr, Val and $H_2N-(CH_2CH_2O)_m-(CH_2)_n-COOH$,

m, n each, independently of one another, denote 0, 1, 2, 3, 4, 5, 6, 7, 8, 9, 10, 11 or 12,

with the proviso that m + n > 0,

where the D and L forms of the optically active amino acid radicals are included,

and physiologically acceptable salts and solvates thereof.

3. Peptidic compounds according to Claim 1 or 2 selected from the group

cyclo(Arg-Gly-Asp-Leu-Asp-Ala-Leu-Arg-Gly-Gly-Gly),

cyclo(Arg-Gly-Asp-Leu-Asp-Gly-Leu-Arg-Gly-Gly-Gly),

cyclo(Arg-Gly-Asp-Leu-D-Ala-Ala-Leu-Arg-Gly-Gly-Gly),

cyclo(Arg-Thr-Asp-Leu-D-Asp-Ala-Leu-Arg-Gly-Gly-Gly),

cyclo(Arg-Gly-Asp-Leu-D-Asp-Ala-Leu-Arg-Abu-Abu),

cyclo(Arg-Gly-Asp-Leu-D-Asp-Ala-Leu-Arg-Aha-Aha),

cyclo(Arg-Gly-Asp-Leu-D-Asp-Ala-Leu-Arg-Aha),

cyclo(Arg-Gly-Asp-Leu-D-Asp-Ala-Leu-Arg-Aee),

cyclo(Arg-Thr-Asp-Leu-D-Asp-Ala-Leu-Arg-Abu-Abu),

cyclo(Arg-Thr-Asp-Leu-D-Asp-Ala-Leu-Arg-$\beta$-Ala),

cyclo(Arg-Gly-Asp-Leu-D-Asp-Ala-Leu-Arg-$\beta$-Ala),

and physiologically acceptable salts and solvates thereof.

4. Peptidic compounds of the formula I according to Claims 1-2 and the compounds according to Claim 3 and physiologically acceptable salts and solvates thereof as medicaments.

5. Medicament according to Claim 4 as inhibitor for combating diseases based on expression and pathological function of $\alpha_v\beta_6$ integrin receptors.

6. Medicament according to Claim 5 for combating thromboses, cardiac infarction, coronary heart diseases, arteriosclerosis, tumours, osteoporosis, fibroses, inflammation, infections, psoriasis and for influencing wound healing processes.

7. Pharmaceutical preparation comprising at least one medicament according to one of Claims 5 and 6 and optionally excipients and/or adjuvants and optionally other active compounds.

8. Use of peptidic compounds according to Claims 1 to 3 and/or physiologically acceptable salts thereof for the preparation of a medicament for combating diseases based on expression and pathological function of $\alpha_v\beta_6$ integrin receptors.

9. Use according to Claim 8 for the preparation of a medicament for combating thromboses, cardiac infarction, coronary heart diseases, arteriosclerosis, tumours, osteoporosis, fibroses, inflammation, infections, psoriasis and for influencing wound healing processes.

**Revendications**

1. Dérivés de peptide de la formule I

cyclo(Arg-$X^1$-Asp-$X^2$-$X^3$-$X^4$-$X^5$-$X^6$-$R^1$)　　　　　I

dans laquelle

$X^1$ représente Ser, Gly ou Thr,
$X^2$ représente Leu, Ile, Nle, Val ou Phe,
$X^3$ représente Asp, Glu, Lys ou Phe,
$X^4$ représente Gly, Ala ou Ser,
$X^5$ représente Leu, Ile, Nle, Val ou Phe,
$X^6$ représente Arg, Har ou Lys,
$R^1$ représente un ou plusieurs radical/radicaux acide $\omega$-aminocarboxylique, où le/les radical/radicaux acide $\omega$-aminocarboxylique présente(nt) une longueur de 500 à 2500 pm,

où les formes D et L des radicaux acide aminé actifs optiquement sont incluses,
et leurs sels et solvates physiologiquement acceptables.

2.  Dérivés de peptide selon la revendication 1 de la formule I

$$\text{cyclo(Arg-}X^1\text{-Asp-}X^2\text{-}X^3\text{-}X^4\text{-}X^5\text{-}X^6\text{-}R^1) \qquad\qquad I$$

dans laquelle

$X^1$ représente Ser, Gly ou Thr,
$X^2$ représente Leu, Ile, Nle, Val ou Phe,
$X^3$ représente Asp, Glu, Lys ou Phe,
$X^4$ représente Gly, Ala ou Ser,
$X^5$ représente Leu, Ile, Nle, Val ou Phe,
$X^6$ représente Arg, Har ou Lys,
$R^1$ représente 1 à 10 acides aminés choisis parmi le groupe Ala, Asn, Asp, Arg, Cys, Gln, Glu, Hcy, His, Hse, Ile, Leu, Lys, Met, Pen, Phe, Pro, Ser, Thr, Trp, Tyr, Val et $H_2N\text{-}(CH_2CH_2O)_m\text{-}(CH_2)_n\text{-}COOH$,
m, n chacun indépendamment de l'autre, représentent 0, 1, 2, 3, 4, 5, 6, 7, 8, 9, 10, 11 ou 12,

étant entendu que m + n > 0,
où les formes D et L des radicaux acide aminé actifs optiquement sont incluses,
et leurs sels et solvates physiologiquement acceptables.

3.  Composés peptidiques selon la revendication 1 ou 2, choisis parmi le groupe

cyclo(Arg-Gly-Asp-Leu-Asp-Ala-Leu-Arg-Gly-Gly-Gly),
cyclo(Arg-Gly-Asp-Leu-Asp-Gly-Leu-Arg-Gly-Gly-Gly),
cyclo(Arg-Gly-Asp-Leu-D-Ala-Ala-Leu-Arg-Gly-Gly-Gly),
cyclo(Arg-Thr-Asp-Leu-D-Asp-Ala-Leu-Arg-Gly-Gly-Gly),
cyclo(Arg-Gly-Asp-Leu-D-Asp-Ala-Leu-Arg-Abu-Abu),
cyclo(Arg-Gly-Asp-Leu-D-Asp-Ala-Leu-Arg-Aha-Aha),
cyclo(Arg-Gly-Asp-Leu-D-Asp-Ala-Leu-Arg-Aha),
cyclo(Arg-Gly-Asp-Leu-D-Asp-Ala-Leu-Arg-Aee),
cyclo(Arg-Thr-Asp-Leu-D-Asp-Ala-Leu-Arg-Abu-Abu),
cyclo(Arg-Thr-Asp-Leu-D-Asp-Ala-Leu-Arg-$\beta$-Ala),
cyclo(Arg-Gly-Asp-Leu-D-Asp-Ala-Leu-Arg-$\beta$-Ala),

et leurs sels et solvates physiologiquement acceptables.

4.  Composés peptidiques de la formule I selon les revendications 1-2 et composés selon la revendication 3 et leurs sels et solvates physiologiquement acceptables en tant que médicaments.

5.  Médicament selon la revendication 4 en tant qu'inhibiteur pour combattre des maladies sur la base d'une expression et d'une fonction pathologique de récepteurs intégrine $\alpha_v\beta_6$.

6.  Médicament selon la revendication 5 pour combattre les thromboses, l'infarctus cardiaque, les maladies coronariennes, l'artériosclérose, les tumeurs, l'ostéoporose, les fibroses, les inflammations, les infections, le psoriasis et pour influencer des processus de cicatrisation des plaies.

**7.** Préparation pharmaceutique comprenant au moins un médicament selon l'une des revendications 5 et 6 et en option, des excipients et/ou des adjuvants et en option, d'autres composés actifs.

**8.** Utilisation de composés peptidiques selon les revendications 1 à 3 et/ou de leurs sels physiologiquement acceptables pour la préparation d'un médicament pour combattre des maladies sur la base d'une expression et d'une fonction pathologique de récepteurs intégrine $\alpha_v\beta_6$.

**9.** Utilisation selon la revendication 8 pour la préparation d'un médicament pour combattre les thromboses, l'infarctus cardiaque, les maladies coronariennes, l'artériosclérose, les tumeurs, l'ostéoporose, les fibroses, les inflammations, les infections, le psoriasis et pour influencer des processus de cicatrisation des plaies.